# EUROPEAN PATENT SPECIFICATION

(11) **EP 3 167 268 B1**
(45) Date of publication and mention of the grant of the patent: **21.02.2024**
(21) Application number: 15818774.0
(22) Date of filing: 10.07.2015
(51) Int. Cl.: G01N 1/16, G01N 1/18, G01N 1/22, G01N 1/26, G01N 1/12, G01N 1/28

(54) **A METHOD OF REMOVING AND DETECTING THE PRESENCE OF SUBSTANCES.**
EINE METHODE ZUR ENTFERNUNG UND ZUM NACHWEIS DES VORHANDENSEINS VON SUBSTANZEN.
UNE MÉTHODE D'ÉLIMINATION ET DE DÉTECTION DE LA PRÉSENCE DE SUBSTANCES.

(30) Priority: 10.07.2014 US 201462022760 P
(43) Date of publication of application: 17.05.2017
(73) Proprietor: Smith, Scott C., Osterville, MA 02655 (US)
(72) Inventor: Smith, Scott C., Osterville, MA 02655 (US)
(74) Representative: CSY Herts
(86) International application number: PCT/US2015/039905
(87) International publication number: WO 2016/007833

(56) References cited:
- US-A- 3 819 514
- US-A- 5 942 440
- US-A1- 2008 203 030
- US-A1- 2011 287 929
- US-A1- 2011 287 929
- US-A1- 2013 240 451
- US-A1- 2013 240 451
- US-B1- 6 237 430
- US-B2- 8 763 478
- US-B2- 8 763 478

## Description

### Background

This disclosure relates to testing water and air for contaminants.

The significance of trace chemicals in our water and air are of increasing interest due to their known and unknown effects on human health, as well as the health of animals and plants, and effects on the ecosystem. Animals and humans that are exposed to contaminants can absorb the chemicals into and through the skin, by breathing the air, and by drinking water including inadvertently while swimming in bodies of water effectively "concentrating" the chemicals into the body or skin. It is desirable to have an accurate and cost efficient method to analyze water and air for the presence of oil and other contaminants, including but not limited to diesel range organics, gasoline range organics, polychlorinated biphenyls (PCBs), fertilizers, pesticides, pharmaceuticals, organometals, metals, metalloids, volatile organic compounds (VOCs), semi-volatile organic compounds (SVOCs) and radioactive materials.

Traditional sampling by collecting and analyzing a split second "grab sample" is useful but has several limitations. Among those limitations is the inability to detect transient contaminants that are discharging sporadically and diffusing through the water column on an irregular basis, and the limited sample size that may contain only undetectable amounts of contaminants that are present at low concentrations and/or below the minimum detection level. Also, grab samples, by their nature, are instantaneous or reflect what is in the water for that split second. As a fish does not swim in the water for a split second and neither does a child, it is desired to have a sampling process that involves accumulation of contaminants over time in the same way that life forms are exposed to contaminants over time throughout the entire water column.

Chemicals and contamination are never in equilibrium in water, let alone water that is constantly flowing with many other variables to consider. Instantaneous/grab sampling reflects what is in the water for a split second, assumes the water being tested is in equilibrium, and does not take into consideration conditions like the constantly changing mixture of fresh water to the chemicals of concern when the grab sample is taken.

Semi-permeable membrane (SPMD) non open-cell foam sampling is the industry standard method for testing accumulation of contaminants due to the ability to concentrate the contaminant over an extended time period, typically 30 days. SPMDs are typically comprised of solid crystalline polyolefins. SPMD devices are described in US Patents 5,098,573 and 5,395,426. However, SPMDs are relatively expensive and as the molecular weight (size) of the contaminant increases, the absorption is reduced or eliminated. Furthermore, with the increase in diluted bitumen ("tar sands"), oil wastewater being used to irrigate crops, and bakken oils, there is a need to monitor and detect the water column and produced water and/or wastewater used in irrigation and/or other applications which cause exposure risk for humans and the environment for drilling fluids, fracking fluids, chemicals used in the transportation of these "newer" oils, heavier oils, high molecular weight contaminants, some of which have specific gravities exceeding that of water, along with other contaminants. Some such contaminants can be difficult and cost prohibitive to monitor with SPMDs. Publications US2013/240451A1, US8763478B2 and US2011/287929A1 disclose related prior art.

### Summary

One subject of this disclosure is an open-cell foam matrix material. The foam material can be produced from a copolymer of ethylene and alkyl acrylate, most preferably ethylene methyl acrylate (EMA). The open-cell structure behaves as the alveoli of the human lungs in that it maximizes surface area which maximizes the efficacy of the open-cell foam's ability to attract oil, chemicals, and related contamination at the molecular level, while repelling water.

Open-cell foam matrix cumulative testing as described herein identifies what is in the water over time. This testing can use (but need not use) EPA testing procedures. This cumulative testing has much less variability than instantaneous grab samples when it comes to potential dilution of the water body being tested and variability within the body of water and various connections to the body of water being tested. It has been established that grab sample testing exhibits such variability that it can lead to non-detects when chemicals of concern are actually present. When considering how animals and humans are exposed to various bodies of water from the bathtub / shower at home to swimming in a lake, pond, or ocean, the concentration index of the open-cell foam matrix cumulative testing appears to be a more accurate representation of the chemicals of concern in the water especially when the body of water is not in equilibrium. Some of the more dangerous chemicals of concern can enter the body through the skin; the skin attracts these chemicals and "concentrates" the chemicals in the human body.

The open-cell foam matrix material can be composed of a relatively amorphous polyolefin elastomer. The open-cell foam structure provides high surface area due to the interconnected structure of the individual cells. The oleophilic nature of the constituent polymer(s) prevents the absorption of water and promotes absorption and adsorption of oils and related substances.

The cumulative environmental indicator device (or detector) is preferably but not necessarily fabricated from a very specific formulation in an open-cell foam. Specifically, this foam is produced from 80 - 100% ethylene acrylate copolymer. Blends of LDPE can be used also. One embodiment / formulation of this open-cell foam is described in US Patent #8,853,289. Another embodiment / formulation of this open-cell foam is described in US patent application publication US 2013/0240451 A1. While 80 - 100% EMA is the preferred formulation of the open-cell foam that is substantially non-polar, what is contemplated herein includes any open-cell foam produced from one or more polymers, such polymers including but not limited to EMA, ethylene vinyl acetate (EVA), ethylene-ethyl acrylate (EEA), ethylene-butyl acrylate (EBA), ethylene propylene diene monomer (EPDM), elastomers, polyolefin elastomers, low density polyethylene (LDPE), linear low density polyethylene (LLDPE), high density polyethylene (HDPE), polypropylene (PP), neoprene, styrene butadiene rubber, ionic co-polymers, other synthetic rubbers, natural rubber, chlorinated polyethylene (CPE), olefin block copolymers, ethylene maleic anhydride copolymer, very low density polyethylene (VLDPE), single site initiated polyolefins, metallocene catalyzed polyolefins, grafted polymers including but not limited to silane and maleic anhydride, styrene-butadiene-styrene copolymers, polyisoprene, and equivalents to any and all of these polymers. The preferred foam density is in the range of from about 16 kg/m³ - 1.0 pound per cubic foot to about 801 kg/m3 - 50.0 pound per cubic foot but the foam can be any density less than the specific gravity of water (998 kg/m³ at 21°C - 62.3 pound per cubic foot at 70°F). The open-cell foam can be extruded or produced in a bun / batch process. The open-cell foam can be crosslinked or non-crosslinked. Also, the open-cell foam can be manufactured using either physical blowing agents or chemical blowing agents. Furthermore, a bio-degradable initiator may be added to the foam so that after use it will degrade over time in a landfill environment when disposed.

The foam is preferably highly oleophilic and substantially non-polar. However, field-testing has shown that the polar methyl acrylate groups of EMA have an ability to attract and absorb polar metals and metalloids (and other polar contaminants) into the open-cell foam matrix / capillary network. Depending upon target contaminants, this disclosure contemplates formulations with increased polarity for detecting radioactive materials / metals along with other chemicals / contaminants that tend to have increased polarity. Its open-cell structure allows for absorption of oils and other lipids into the network cell structure of the foam. Preferred but non-limiting cell size ranges are from about 0.1 mm to about 3.5 mm. Water is repelled. It has been demonstrated that this selective absorption of lipids makes the foam desirable for use as an oil and related chemicals indicator/detector in bodies of water.

A cumulative environmental indicator device herein can comprise one or more separate portions (e.g., pieces or strips) of the subject foam that are held in a water column, at one depth or at multiple depths. The foam is less dense than water so strip(s) will extend upward in the water column from the location where they are held/anchored. Each strip can extend over some or all of the depth (i.e., the height of the water column). The foam is left in place for an extended period of time, longer than is accomplished with a grab sample. The time can be any amount of time - seconds, minutes, hours, days, weeks or months, depending on the situation and desired results. The foam absorbs and adsorbs oils and other contaminants. These contaminants are accumulated by the foam. The foam is then removed and tested for the presence and concentration of contaminants. Since the indicator spans different depths, the results can determine the presence and concentration of one or more contaminants at different depths of the water column, from the surface to the bottom, as desired. Also, as the foam portions can be placed at different locations in a body of water (as well as at different depths if desired), they can detect the movement of plumes of contamination through the water.

The indicator can be fabricated into a number of structures to suit the application of the indicator. One preferred structure is an assembly of strips, typically 1.3-1.9cm × 1.3-1.9cm × 30-46 cm (0.5-0.75 inch × 0.5-0.75 inch × 12 - 18 inches). The strips can be fastened together tightly at the center to form a structure with multiple "fingers" or "blades." This structure exposes a large surface area to the environment, and allows flow through (between the fingers of) the indicator. These indicators are then fastened to a rope line or similar tether with a weight at one end, and are submerged into the water body, leaving indicators at various depths. Another alternative is to have strips of the foam that are anchored to the bottom and extend to the surface, over the entire water column; this is called "eelgrass" since it looks like eelgrass that grows in the ocean. Other forms can include strips and smaller cubes and pieces in other shapes. Another form includes a structure with multiple smaller fingers sized such that the structure can be cast (either weighted or not weighted) into the water or water column with a fishing rod, and retrieved. Placing the open-cell foam material into the body of water may comprise dragging the open-cell foam material behind a boat that moves through the water, or floating the open-cell material on the surface of the water, or coupling the open-cell foam material to a dock, or placing the open-cell foam material in a bathtub or sink. Any form can be placed anywhere in the water column and/or on the surface of the water. Smaller pieces can be held in place in nets or other containers with holes to allow water flow. Such containers can be made of plexiglass or other materials.

The indicator can be designed to monitor the water for accumulation of chemicals over time. The indicator can also be used to remove the chemicals from the water. Indicators can be in the forms of eelgrass, cubes, pieces, and/or strips, and can be but need not be contained in a cylinder or net. These forms can be floating on the surface or suspended and/or submerged in the water column using anchors.

Indicators that are fabricated from the described foam have demonstrated the ability to detect and remove lipids, oils, metals, organometals, metalloids, PCBs, the full range of SVOCs, the full range of VOCs, and other substances in bays and harbors as well as open bodies of water and fresh water lakes, rivers, and streams, and in residences (e.g., bathtubs and sinks).

Advantages of this indicator are its efficient cost, ease of deployment, durability during deployment and in use, and ability to collect large samples over an extended time period.

Upon retrieval of the indicator from the water or air, the open-cell foam can be placed into a sealed container and sent to a qualified lab to test the open-cell foam matrix with various EPA and other testing methods.

Furthermore, the foam can remove and detect food sources for what is known as Blue-Green Algae / Cyanobacteria, including phosphorous. By deploying various designs of fabricated open-cell foam it is contemplated that phosphorous can be effectively removed to assist in the prevention of Blue-Green Algae / Cyanobacteria blooms. One such embodiment is a solar powered filtration system that uses open-cell foam pieces (e.g., cubes) in a flow-through container (filter) such as a cylindrical structure that is scalable to the size of the body of water. A pump can be used to flow water through the filter. The pump can be but need not be solar or battery powered. The filter media (foam pieces) can be changed as necessary. This filtration system can be used to remove all other substances that are referenced in this disclosure, in various bodies of water.

In one aspect, a method of removing and detecting the presence of substances from at least one of a body of water and the air includes placing into the body of water or into the air an open-cell foam material, removing separate portions of the open-cell foam material from the water or air at different exposure times after the open-cell foam material was placed into the water or air, and determining the presence in the removed separate portions of one or more substances that were removed from the water or air by the open-cell foam material.

Embodiments may include one of the following features, or any combination thereof. The open-cell foam material may be substantially non-polar. The open-cell foam material may comprise a cross-linked copolymer of ethylene and alkyl acrylate. The open-cell foam material may comprise a cross-linked blend of a copolymer of ethylene and alkyl acrylate, and optionally one or more other polymers selected from the group of polymers including but not limited to LDPE, LLDPE, ionic copolymers, natural rubber, synthetic rubber, elastomers, and HDPE. The alkyl acrylate may comprise methyl acrylate or ethyl acrylate, for example.

Embodiments may include one of the following features, or any combination thereof. The open-cell foam material may comprise one or more of EMA, EVA, EEA, EBA, LDPE, LLDPE, HDPE, VLDPE, PP, natural rubber, EPDM, synthetic rubber, elastomer, polyolefin elastomer, polypropylene, CPE, olefin block copolymers, ethylene maleic anhydride copolymer, singe site initiated polyolefins, metallocene catalyzed polyolefins, grafted polymers including but not limited silane and maleic anhydride, styrene-butadiene-styrene copolymers, polyisoprene, and equivalents to any and all of these polymers and blends thereof. The open-cell foam material may comprise a copolymer comprising a polar component. The substances that are removed may be selected from the group of substances consisting of oil, diesel range organics, gasoline range organics, drilling fluids, biocides, glutaraldehyde, metals, organometals, metalloids, VOCs, SVOCs, pesticides, PCBs, radioactive substances, fertilizers, solvents, human waste, pharmaceuticals, and components thereof.

Embodiments may include one of the following features, or any combination thereof. Placing the open-cell foam material may comprise suspending a plurality of separate structures at different levels through a height of a water column. Placing the open-cell foam material may further comprise placing a plurality of separate structures at different locations in the body of water. Placing the open-cell foam material may further comprise floating a structure at least partially on the surface of the water. Placing the open-cell foam material into the body of water may comprise casting the open-cell foam material into the water with a fishing rod. Placing the open-cell foam material into the body of water may comprise dragging the open-cell foam material behind a boat that moves through the water, or floating the open-cell material on the surface of the water, or coupling the open-cell foam material to a dock, or placing the open-cell foam material in a bathtub or sink. Removing separate portions of the open-cell foam material from the water or air at different exposure times after the open-cell foam material was placed into the water or air, can take place at more than one time over an exposure time of at least eight hours, or at least one day.

Embodiments may include one of the following features, or any combination thereof. The open-cell foam material may comprise a plurality of separate structures selected from the group of structures consisting of strips, strips that are longer than a height of a water column, cubes, and small pieces. The separate structures may be held in place by one or more of an anchor, a weight, a netting, and a container with openings to allow the flow of water therethrough.

In another aspect, a method of removing and detecting the presence of substances from at least one of a body of water and the air, includes suspending a plurality of separate structures comprising an open-cell foam material in the form of strips, strips that are longer than a height of a water column, cubes, and small pieces, at different levels through a height of a water column of the body of water and at different locations in the body of water, removing separate portions of the structures from the water at different exposure times after the structures were placed into the water, and determining the presence in the removed separate portions of one or more substances that were removed from the water by the structures, wherein the substances are selected from the group of substances consisting of oil, diesel range organics, gasoline range organics, drilling fluids, biocides, glutaraldehyde, metals, organometals, metalloids, VOCs, SVOCs, pesticides, PCBs, fertilizers, solvents, human waste, pharmaceuticals, and components thereof. The open-cell foam material may comprise one or more of EMA, EVA, EEA, EBA, LDPE, LLDPE, HDPE, VLDPE, PP, natural rubber, EPDM, synthetic rubber, elastomer, polyolefin elastomer, polypropylene, CPE, olefin block copolymers, ethylene maleic anhydride copolymer, singe site initiated polyolefins, metallocene catalyzed polyolefins, grafted polymers including but not limited silane and maleic anhydride, styrene-butadiene-styrene copolymers, polyisoprene, and equivalents to any and all of these polymers and blends thereof.

In one aspect of the invention when testing air, the open-cell foam can be moved through the air (e.g., flown through the air with a drone or helicopter) and then tested for contaminants as described elsewhere herein. Or the foam can be placed in the air at one or more locations on the ground and/or at different heights above the ground, and left for desired amounts of time (such as described herein relative to water testing).

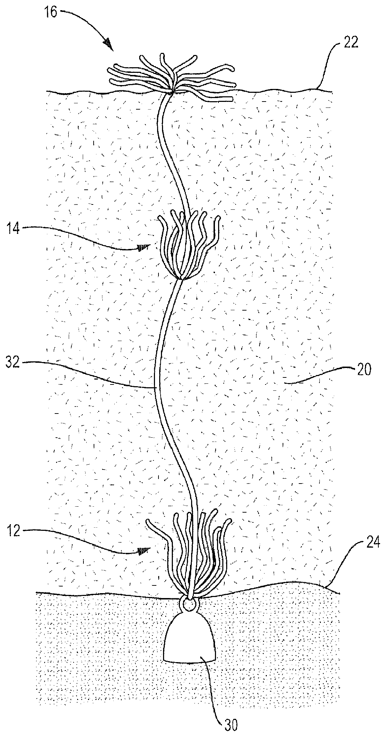

### Brief Description of the Drawing

The drawing depicts one non-limiting example of the placement of open-cell foam material into a body of water.

### Description of Examples

Methods of removing and detecting the presence of substances (such as, but not limited to, contaminants) from a body of water or the air are disclosed. As a first step, an open-cell foam material can be placed into the body of water or into the air. The placement can be at one or more locations in the body of water or air, and at one or more depths or heights in the body of water or in the air. After desired exposure times, separate portions of the open-cell foam material are removed from the water or air. The presence in the removed separate portions of one or more substances that were removed from the water or air by the open-cell foam material are then determined, typically by standard EPA testing procedures.

The methods are effective both to determine the presence of substances such as contaminants in the water or air, and also to remove such substances from the water or air. The methods thus can be used for contaminant detection and/or filtration/remediation.

The drawing depicts three groups of strips or "blades" of open-cell foam material 12, 14 and 16. Each group has multiple strips that are held together at about their centers, The groups are fastened to a line 32 that is held on the bottom 24 of water body 20 by weight or anchor 30. In this example group 16 floats on the water surface 22, while groups 12 and 14 are held at different depths below the surface. This disclosure allows for the placement of open-cell foam material at any one or more heights of a body of water and/or the air, and at one or more locations in the body of water or air. Various non-limiting methods of exposing the open-cell material to water or air are described herein; any such method can be used as desired or as necessary ddepending on the body of water and/or the testing regime that is desired under the circumstances.

After desired exposure times, separate portions of the foam material are removed from the water or air. This can be done by clipping or cutting a piece of foam, or removing an entire group or other portion or separate piece of foam, for example. The exposure times can be from seconds to minutes to hours to days to weeks to months, depending on the particular testing regime. Since the foam absorbs and adsorbs certain materials (described elsewhere herein), the removed portions of the foam can be tested for particular substances. The foam can act as an accumulator for these substances. Also, the different locations and different exposure times allow for a tailored review of contaminants, their locations and their movement within the water or air.

The subject material has been used in extensive testing of various bodies of water, both salt and fresh water, including open bodies of water, rivers, streams and irrigation canals. Open-cell foam material has been shown to remove from water at least the following types of contaminants. Non-limiting examples of each type of contaminant are also listed; these examples illustrate contaminants that are in each type or group, but are not limiting.
- Metals and Metalloids: examples include but are not limited to Arsenic, Barium, Boron, Cadmium, Cooper, Lead, Manganese, Mercury, Nickel, Phosphorus, Vanadium, Yttrium.
- Volatile Organic Compounds ("VOCs"): examples include but not limited to Tetrachloroethane, Trimethylbenzene, Butanone, Acetone, Benzene, Ethylbenzene, Methylene Chloride, Xylene, Toluene.
- Semi Volatile Organic Compounds ("SVOCs"): examples include but not limited to Naphthalene, Anthracene, Chrysene, Fluorene, Hexachlorobenzene, Nitrobenzene, Pyrene, Dimethyl Phthalate.
- Drilling / Transportation / biocide fluids: examples include but not limited to Glutaraldehyde, Benzene compounds, Toluene compounds, Xylene compounds.
- Coal related fluids: examples include but not limited to MCHM (4-Methylcyclohexanemethanol).
- Fluids / Solvents that for example leach from landfills into peoples homes: examples include but not limited to 1,4 Dioxane.
- Radioactive materials: examples include but not limited to: Strontium, Uranium, Yttrium, Rhenium.

### Test Methods and Results

Results of water testing using an environmental indicator made from an open-cell foam material of a type disclosed in the US Patent (e.g., 100% EMA), were set forth in the Appendices of the priority US Provisional Patent Application serial number 62/022,760 filed on July 10, 2014. Some of that data is set forth and summarized below.

The testing methods used by certified third party laboratories include but are not limited to: EPA SW8015B, EPA SW7471A, EPA SW6010B, EPA SW8270C, EPA SW8260B, IH-004, ALS Method 8270 (for MCHM), and EPA 1664 (modified) for oil and grease.

One test set detected low levels of PCBs in the harbor at New Bedford, Massachusetts. Data was included in the appendices.

In Knapp Creek, PA, Oak Glen Nature Preserve, OH, Lynchburg, VA and Aliceville, AL, it was discovered that the cumulative environmental indicator detected and removed organometals, metals, metalloids, VOCs, and SVOCs, including chemicals like 4-methylcyclohexanemethanol (MCHM), which was spilled in Charleston, WV, USA. See third party testing results (in the appendix), which illustrates the accumulation over time into the open-cell EMA foamed matrix, mimicking environmental uptake by living organisms. It is of note that arsenic, barium, lead, and mercury were detected in Knapp Creek. There were harsh winter weather conditions and the open-cell EMA retained and absorbed these metals and related compounds.

Results of uses of the environmental indicator in water are disclosed in the appendices I-V that are incorporated herein. A brief discussion of those appendices follows.

Appendix I is a table (printed on two pages) that includes a comparison of the environmental indicator to a grab sample taken from the surface of the water (at 8:30 AM when the test began) after a coal ash spill in the Dan river in Eden, NC. See tables 1A and 1B below for the data. The indicators (environmental indicators or "EI") were in three different forms: one was anchored with strips at the bottom, middle and top of the water column (such as in the drawing), a second was in the form of floating eelgrass, and the third was in the form of a mitt made of the material that was submerged in the water column. These various forms of indicators were removed at three different times to show exposure or accumulation of contaminants over time, and the samples were sent for analysis to a third-party lab. Organometals, metals, metalloids and SVOCs were detected at different times and different heights in the water column. Samples were taken with instantaneous grab samples along with open-cell foam cumulative samples at various exposure times. The grab sample showed non-detects (N/D) for everything with the exception of iron. The open-cell foam cumulative samples at various exposure times detected the presence of metals and SVOCs. Furthermore, the open-cell foam cumulative samples (indicators) illustrated the importance of sampling the entire water column at various depths. For example, the bottom indicator detected the presence of manganese, phosphorus, and titanium while the middle and top indicators showed non-detects. Also, the open-cell foam mitt that was exposed to the water near shore from top to bottom in about (61 cm - 2 feet) of water for 3 minutes (right next to where the instantaneous grab sample was taken) detected the presence of iron, manganese, phosphorous, titanium, and SVOCs while the grab sample only detected the presence of iron.

Appendix II is a table (printed on three pages) that includes results taken after a bakken oil spill in Aliceville, AL. A variety of forms of the indicator were placed into the water in and around the site of this bakken oil train explosion and a variety of residual SVOCs and VOCs were detected leaching out of the soil and into the wetlands approximately three months after the oil train explosion. Pure oil samples were taken as leaching from the soil and these were used as a baseline to compare to what the indicators absorbed in the nearby surrounding waters from the wetlands.

Appendix III is a table (printed on one page) that includes results taken from the banks of the James River in Lynchburg, VA. These samples were collected from the river bank of the James River after a bakken oil train exploded. An indicator was placed directly into the contaminated river bank and was then placed into a sealed glass jar and sent to a third party lab. A variety of SVOCs, VOCs, and organometals, metals, and metalloids were detected that were consistent with prior bakken oil train explosions. Appendices III a (four pages) and III b (four pages) are publicly available results as posted by the James River Association and Arcadis. The James River Association and Arcadis tests showed non-detects which includes but is not limited to the following metals and oil compounds while the environmental indicators detected these metals and oil compounds: barium, chromium, nickel, phosphorus, vanadium, acetone, trimethylbenzene, xylene and napthalene.

Appendix IV is a table (printed on two pages) that includes results from various open-cell foam cumulative samples were taken from the water column after an oil spill in Galveston, TX. See Table 4 for the data. Eelgrass indicators and submerged indicators were placed into the water. Of four samples taken (3 surface (two with eelgrass and one with an environmental indicator) and 1 middle of water column), the middle indicator detected chromium, cobalt, and lead were detected but not detected in the others. Also, vanadium was detected on the surface eelgrass after exposure of approximately 43 hours but had a non-detect after 20 hours. Vanadium was detected by the middle indicator in the middle of the water column after 20 hours (again there was a non-detect on the surface after 20 hours).

Appendix V is a table (printed on one page) that includes results taken from three locations in Nantucket harbor, Nantucket, MA. Environmental indicators were placed in the entire water column and retrieved at different lengths of time to monitor exposure over time. When retrieved the indicators were placed in plastic and glass sealed containers for third party lab testing. At the boat dock, acetone was detected in the middle of the water column but not at the top or bottom. At the town pier, vanadium was detected at the bottom of the water column but not at the middle of the water column.

Various open-cell foam cumulative samples were taken in the Cawelo water district in Kern County, CA where wastewater from oil drilling and/or refining operations is filtered and diluted with fresh water and then feed to a canal system that is used to irrigate crops. Baseline oil-water as it comes out of the ground was tested along with downstream water throughout the canal system. See tables 2A-2D for data (blank cells in the tables are non-detects). TPH stands for total petroleum hydrocarbons, baseline oil is the subject oil that is leaking or spilling in its raw form, irr pond is an irrigation pond, Poso Creek was baseline oil-water coming right out of the ground in its raw state. The post-filtration dilution locations were various locations throughout the irrigation canal system in the Cawelo Water District of Kern County, California, USA. The presence of oil and VOCs were found downstream that matched the chemicals found in the baseline oil-water mixture. Also, there were various non-detects throughout the canal system which confirmed that the water is not in equilibrium and the importance of multiple sample points with the ability to detect chemicals over time / exposure.

Separetely from the irrigation water in the Cawelo water district and in another part of Kern County, various open-cell foam cumulative samples were taken from the surface of oil wastewater discharge points in unlined pits. The data gathered (presented in Table 3) illustrates that even in this relatively confined water discharge system that there is no equilibrium of contaminants in water. All samples were exposed to the surface water for 30 minutes. Metals, metalloids, and VOCs showed various detects and non-detects (blank table cells), along with variation in concentration levels.

The fact that testing with the subject material has had non-detects and detects for chemicals of concern in the same bodies of water is not only expected but is absolute proof that chemicals / contamination are not in equilibrium in water. Thus, using an accumulator as described herein is highly beneficial for determining actual contaminants in water.

**Table 1A**

| **Exposure Time** | 3 Minutes | Instantaneous - 1 Second | 9 Hours | 3 Hours / Surface | 3 Hours |
|---|---|---|---|---|---|
| **Sample** | Mitt | Grab Sample | El Bot | El Float Kayak | El Mid |
| **Metals** | Units (ppm) | Units (ppm) | Units (ppm) | Units (ppm) | Units (Ppm) |
| Iron | 830 | 0.81 | 480 | 220 | 110 |
| Manganese | 11 | N/D | N/D | 33 | N/D |
| Phosphorus | 17 | N/D | 13 | 13 | N/D |
| Titanium | 47 | N/D | 17 | N/D | N/D |

| **SVOC's** | Units (ppb) | Units (ppb) | Units (ppb) | Units (ppb) | Units (ppb) |
|---|---|---|---|---|---|
| Bis(2-ethylhexl) phthalate | 640 | N/D | 2200 | 270 | 230 |
| Di-n-octyl phthalate | N/D | N/D | 340 | N/D | 720 |

**Table 1B**

| **Exposure Time** | 3 Hours | 3 Hours | 6.5 Hours | 6.5 Hours | 6.5 Hours |
|---|---|---|---|---|---|
| **Sample** | El Top | El Bot | El Top | El Mid | El Bot |
| **Metals** | Units (ppm) | Units (ppm) | Units (ppm) | Units (ppm) | Units (ppm) |
| Iron | 110 | 210 | 120 | 220 | 310 |
| Manganese | N/D | N/D | N/D | N/D | 5.4 |
| Phosphorus | N/D | N/D | N/D | N/D | 15 |
| Titanium | N/D | N/D | N/D | N/D | 12 |

| **SVOC's** | Units (ppb) | Units (ppb) | Units (ppb) | Units (ppb) | Units (ppb) |
|---|---|---|---|---|---|
| Bis(2-ethylhexl) phthalate | 210 | 120 | 810 | 1500 | 430 |
| Di-n-octyl phtalate | 270 | 550 | 550 | N/D | N/D |

**Table 2A**

| Exposure Time | Instant | Instant | 17 Hours | 17 Hours |
|---|---|---|---|---|
| Location | Poso Creek Oil Field | Poso Creek Oil Field | Post Dilution / Water Treatment | Post Dilution / Water Treatment |
| Sample | Baseline Oil w Mitt | Baseline Oil w/ El | Canal Top El | Canal Bottom El |
| Oil | PPM | PPM | PPM | PPM |
| TPH C20-C34 | 240000 | 480000 | 940 | 340 |
| | | | | |
| VOC's | PPB | PPB | PPB | PPB |
| Acetone | 440 | 530 | 57 | |
| 1,2,4-Trimethlybenzene | 400 | 160 | | |
| 1,3,5-Trimethlybenzene | 110 | 52 | | |
| m,p-Xylene | 120 | 66 | | |
| o-Xylene | 70 | | | |
| Methylene Chloride | 89 | 82 | 26 | |

**Table 2B**

| Exposure Time | 30 Minutes | 30 Minutes | 5 Hours | 5 Hours |
|---|---|---|---|---|
| Location | Post Dilution / Water Treatment | Post Dilution / Water Treatment | Post Dilution / Water Treatment | Post Dilution / Water Treatment |
| Sample | In Pond Eelgrass | Unlined Pond El | Canal Top El | Canal Bottom El |
| Oil | PPM | PPM | PPM | PPM |
| TPH C20-C34 | 1300 | 180 | 270 | 130 |
| | | | | |
| VOC's | PPB | PPB | PPB | PPB |
| Acetone | | | 79 | |
| 1,2,4-Trimethlybenzene | | | | |
| 1,3,5-Trimethlybenzene | | | | |
| m,p-Xylene | | | | |
| o-Xylene | | | | |
| Methylene Chloride | | 32 | 31 | 30 |

**Table 2C**

| Exposure Time | 7 Months | 30 Minutes | 30 Minutes |
|---|---|---|---|
| Location | Post Dilution / Water Treatment | Post Dilution / Water Treatment | Post Dilution / Water Treatment |
| Sample | Canal Top Indicator A | Canal Top Indicator | Canal Middle Indicator |
| Oil | PPM | PPM | PPM |
| TPH C20-C34 | 230 | | |
| | | | |
| VOC's | PPB | PPB | PPB |
| Acetone | | | |
| 1,2,4-Trimethlybenzene | | | |
| 1,3,5-Trimethlybenzene | | | |
| m,p-Xylene | | | |
| o-Xylene | | | |
| Methylene Chloride | | 56 | 44 |

**Table 2D**

| Exposure Time | 30 Minutes | 44 Hours |
|---|---|---|
| Location | Post Dilution / Water Treatment | Post Dilution / Water Treatment |
| Sample | Canal Bottom Indicator | Canal Eelgrass |
| Oil | PPM | PPM |
| TPH C20-C34 | | |
| | | |
| VOC's | PPB | PPB |
| Acetone | | |
| 1,2,4-Trimethlybenzene | | |
| 1,3,5-Trimethlybenzene | | |
| m,p-Xylene | | |
| o-Xylene | | |
| Methylene Chloride | 48 | 26 |

**Table 3**

| Exposure Time | 30 Minutes | 30 Minutes | 30 Minutes |
|---|---|---|---|
| Location | Kern County | Kern County | Kern County |
| Sample | Eelgrass A | Indicator | Eelgrass B |
| Oil | PPM | PPM | PPM |
| TPH C10-C20 | 320 | 650 | 9100 |
| TPH C20-C34 | 670 | 1900 | 27000 |
| Metals | PPM | PPM | PPM |
| Barium | 11 | 10 | |
| Boron | 31 | 42 | 14 |
| Copper | | 25 | |
| Iron | 220 | | |
| Phosphorous | 12 | | |
| Sodium | 2000 | 3000 | |

| VOC's | PPB | PPB | PPB |
|---|---|---|---|
| Acetone | 250 | 560 | 90 |
| 1,2,4-Trimethlybenzene | 65 | 46 | 240 |
| 1,3,5-Trimethlbenzene | | | 69 |
| 2-Butanone | 36 | 75 | |
| Benzene | 76 | 360 | 31 |
| Carbon Disulfide | | | 31 |
| m,p-Xylene | 130 | 180 | 330 |
| n-Butylbenzene | | | |
| o-Xylene | 64 | 97 | 170 |
| Ethylbenzene | 36 | 63 | 120 |
| Methylene Chloride | 27 | | |
| Isopropylbenzene | | | 30 |
| Napthalene | | | 40 |
| N-Propylbenzene | | | 58 |
| Toluene | 200 | 600 | 240 |

**Table 4**

| Exposure Time | 20 Hours | 19 Hours | 19 Hours | 19 Hours |
|---|---|---|---|---|
| Sample | Eelgrass | Eelgrass | Indicator | Indicator / |
| | Surface | Surface | Surface / Top | Middle |
| Metals | PPM | PPM | PPM | PPM |
| Aluminum | 16 | 221 | 76.4 | 226 |
| Barium | 1.57 | 5.29 | 2.31 | 2.97 |
| Calcium | 4960 | 4640 | 3580 | 4600 |
| Chromium | N/D | N/D | N/D | 0.594 |
| Cobalt | N/D | N/D | N/D | 0.641 |
| Lead | N/D | N/D | N/D | 0.69 |
| Magnesium | 886 | 1100 | 943 | 1140 |
| Manganese | 4.39 | 49.8 | 17 | 49.8 |
| Molybdenum | 0.514 | N/D | N/D | 0.725 |
| Iron | 54.3 | 0.677 | 248 | 857 |
| Sodium | 4330 | 5160 | 5070 | 5300 |
| Tin | 3.27 | 4.53 | 2.53 | N/D |
| Titanium | 0.493 | 4.67 | 1.66 | 4.07 |
| Vanadium | N/D | 0.776 | N/D | 0.757 |
| Zinc | 55.1 | 35.4 | 27.5 | 33.4 |

## Claims

1. A method of removing and detecting the presence of substances from at least one of a body of water or the air, comprising:
placing an open-cell foam material into a particular location of the body of water or into a particular location of the air; and then
after a first exposure time in the body of water or the air, removing a first portion of the open-cell foam material from the water or air, and leaving the remainder of the open-cell foam material in the particular location; and then
after a second exposure time in the body or water or air that is longer than the first exposure time, removing a second, separate, portion of the open-cell foam material from the water or air; and then
determining the presence in the removed first and second separate portions, of one or more substances that were removed from the water or air by the open-cell foam material.

2. The method of claim 1, wherein the open-cell foam material is substantially non-polar.

3. The method of any preceding claim, wherein the open-cell foam material comprises a cross-linked blend of a copolymer of ethylene and alkyl acrylate, and optionally one or more other polymers selected from the group of polymers consisting of low density polyethylene (LDPE), linear low density polyethylene (LLDPE), ionic copolymers, natural rubber, synthetic rubber, elastomers, and high density polyethylene (HDPE).

4. The method of claim 3, wherein the alkyl acrylate comprises at least one of methyl acrylate and ethyl acrylate.

5. The method of any preceding claim, wherein the open-cell foam material comprises at least one of ethylene methyl acrylate (EMA), ethylene vinyl acetate (EVA), ethylene-ethyl acrylate (EEA), ethylene-butyl acrylate (EBA), low density polyethylene (LDPE), linear low density polyethylene (LLDPE), very low density polyethylene (VLDPE), high density polyethylene (HDPE), polypropylene (PP), natural rubber, ethylene propylene diene monomer (EPDM), synthetic rubber, chlorinated polyethylene (CPE), olefin block copolymers, ethylene maleic anhydride copolymer, single site initiated polyolefins, metallocene catalyzed polyolefins, grafted polymers including but not limited to silane and maleic anhydride, styrene-butadiene-styrene copolymers, polyisoprene, and equivalents and blends thereof.

6. The method of any preceding claim, wherein the substances are selected from the group of substances consisting of oil, diesel range organics, gasoline range organics, drilling fluids, biocides, glutaraldehyde, metals, organometals, metalloids, volatile organic compounds (VOCs), semi- volatile organic compounds (SVOCs), pesticides, polychlorinated biphenyls (PCBs), fertilizers, solvents, human waste, pharmaceuticals, radioactive materials, and components thereof.

7. The method of any preceding claim, wherein the open-cell foam material comprises a plurality of separate structures selected from the group of structures consisting of strips, cubes, and small pieces, wherein the separate structures are held in place by one or more of an anchor, a weight, a netting, and a container with openings to allow the flow of water therethrough.

8. The method of any preceding claim, wherein placing the open-cell foam material comprises suspending a plurality of separate structures at different levels through a height of a water column, including floating a structure at least partially on the surface of the water.

9. The method of any preceding claim, wherein the second exposure time is at least eight hours.

10. The method of claim 1 wherein placing the open-cell foam material comprises casting the open-cell foam material into the body of water with a fishing rod.

11. The method of claim 1 wherein placing the open-cell foam material comprises dragging the open-cell foam material behind a boat that moves through the body of water, or floating the open-cell material on the surface of the body of water, or coupling the open-cell foam material to a dock, or placing the open-cell foam material in a bathtub or sink.

## Patentansprüche

1. Methode zur Entfernung und zum Nachweis des Vorhandenseins von Substanzen aus mindestens einem von einem Gewässer oder der Luft, umfassend:
Platzieren eines offenzelligen Schaumstoffmaterials an einer jeweiligen Stelle des Gewässers oder an einer jeweiligen Stelle in der Luft; und dann
nach einer ersten Expositionszeit in dem Gewässer oder der Luft, Entfernen eines ersten Abschnitts des offenzelligen Schaumstoffmaterials aus dem Wasser oder der Luft und Belassen des restlichen offenzelligen Schaumstoffmaterials an der jeweiligen Stelle; und dann
nach einer zweiten Expositionszeit in dem Gewässer oder in der Luft, die länger ist als die erste Expositionszeit, Entfernen eines zweiten, separaten Abschnitts des offenzelligen Schaumstoffmaterials aus dem Wasser oder der Luft; und dann
Bestimmen des Vorhandenseins einer oder mehrerer Substanzen, die durch das offenzellige Schaumstoffmaterial aus dem Wasser oder der Luft entfernt wurden, in dem entfernten ersten und zweiten separaten Abschnitt.

2. Methode nach Anspruch 1, wobei das offenzellige Schaumstoffmaterial im Wesentlichen unpolar ist.

3. Methode nach einem vorhergehenden Anspruch, wobei das offenzellige Schaumstoffmaterial eine vernetzte Mischung aus einem Copolymer aus Ethylen und Alkylacrylat und optional einem oder mehreren anderen Polymeren umfasst, die ausgewählt sind aus der Gruppe von Polymeren, bestehend aus Polyethylen niedriger Dichte (LDPE), linearem Polyethylen niedriger Dichte (LLDPE), ionischen Copolymeren, Naturkautschuk, synthetischem Kautschuk, Elastomeren und Polyethylen hoher Dichte (HDPE).

4. Methode nach Anspruch 3, wobei das Alkylacrylat mindestens eines von Methylacrylat und Ethylacrylat umfasst.

5. Methode nach einem vorhergehenden Anspruch, wobei das offenzellige Schaumstoffmaterial mindestens eines von Ethylenmethylacrylat (EMA), Ethylenvinylacetat (EVA), Ethylenethylacrylat (EEA), Ethylenbutylacrylat (EBA), Polyethylen niedriger Dichte (LDPE), linearem Polyethylen niedriger Dichte (LLDPE), Polyethylen sehr niedriger Dichte (VLDPE), Polyethylen hoher Dichte (HDPE), Polypropylen (PP), Naturkautschuk, Ethylen-Propylen-Dien-Monomer (EPDM), synthetischem Kautschuk, chloriertem Polyethylen (CPE), Olefin-Block-Copolymeren, Ethylen-Maleinsäureanhydrid-Copolymer, durch eine einzelne Stelle initiierten Polyolefinen, metallocenkatalysierten Polyolefinen, gepfropften Polymeren, einschließlich, aber nicht beschränkt auf Silan und Maleinsäureanhydrid, Styrol-Butadien-Styrol-Copolymeren, Polyisopren sowie Entsprechungen und Mischungen davon umfasst.

6. Methode nach einem vorhergehenden Anspruch, wobei die Substanzen ausgewählt sind aus der Gruppe von Substanzen, bestehend aus Öl, organischen Stoffen aus dem Dieselbereich, organischen Stoffen aus dem Benzinbereich, Bohrflüssigkeiten, Bioziden, Glutaraldehyd, Metallen, Organometallen, Metalloiden, flüchtigen organischen Verbindungen (VOCs), halbflüchtigen organischen Verbindungen (SVOCs), Pestiziden, polychlorierten Biphenylen (PCBs), Düngemitteln, Lösungsmitteln, menschlichen Abfällen, Pharmazeutika, radioaktiven Materialien und Komponenten davon.

7. Methode nach einem vorhergehenden Anspruch, wobei das offenzellige Schaumstoffmaterial eine Vielzahl von separaten Strukturen umfasst, die ausgewählt sind aus der Gruppe von Strukturen, bestehend aus Streifen, Würfeln und kleinen Stücken, wobei die separaten Strukturen durch einen oder mehrere Anker, ein Gewicht, ein Netz und einen Behälter mit Öffnungen, die den Durchfluss von Wasser dort hindurch ermöglichen, an Ort und Stelle gehalten werden.

8. Methode nach einem vorhergehenden Anspruch, wobei ein Platzieren des offenzelligen Schaumstoffmaterials ein Aufhängen einer Vielzahl separater Strukturen auf verschiedenen Ebenen über einer Höhe einer Wassersäule umfasst, einschließlich Schweben einer Struktur mindestens teilweise auf der Oberfläche des Wassers.

9. Methode nach einem vorhergehenden Anspruch, wobei die zweite Expositionszeit mindestens acht Stunden ist.

10. Methode nach Anspruch 1, wobei ein Platzieren des offenzelligen Schaumstoffmaterials ein Werfen des offenzelligen Schaumstoffmaterial mit einer Angelrute in das Gewässer umfasst.

11. Methode nach Anspruch 1, wobei ein Platzieren des offenzelligen Schaumstoffmaterials ein Ziehen des offenzelligen Schaumstoffmaterials hinter einem Boot, das sich durch das Gewässer bewegt, oder ein Schweben des offenzelligen Schaumstoffmaterials auf der Oberfläche des Gewässers oder ein Koppeln des offenzelligen Schaumstoffmaterials mit einem Dock oder ein Platzieren des offenzelligen Schaumstoffmaterials in einer Badewanne oder einem Waschbecken umfasst.

## Revendications

1. Procédé de retrait et de détection de la présence de substances d'au moins un élément parmi un corps d'eau ou l'air, comprenant :
le placement d'un matériau en mousse à alvéoles ouvertes dans un emplacement particulier du corps d'eau ou dans un emplacement particulier de l'air ; puis
après un premier temps d'exposition dans le corps d'eau ou l'air, le retrait d'une première partie du matériau en mousse à alvéoles ouvertes de l'eau ou de l'air, et le fait de laisser le reste du matériau en mousse à alvéoles ouvertes dans l'emplacement particulier ; puis
après un second temps d'exposition dans le corps d'eau ou l'air qui est plus long que le premier temps d'exposition, le retrait d'une seconde partie distincte du matériau en mousse à alvéoles ouvertes de l'eau ou de l'air ; puis
la détermination de la présence dans les première partie et seconde partie distincte retirées, d'une ou plusieurs substances qui ont été retirées de l'eau ou de l'air par le matériau en mousse à alvéoles ouvertes.

2. Procédé selon la revendication 1, ledit matériau en mousse à alvéoles ouvertes étant sensiblement non polaire.

3. Procédé selon l'une quelconque des revendications précédentes, ledit matériau en mousse à alvéoles ouvertes comprenant un mélange réticulé d'un copolymère d'éthylène et d'acrylate d'alkyle, et éventuellement un ou plusieurs autres polymères choisis dans le groupe des polymères constitués de polyéthylène basse densité (PEBD), de polyéthylène à basse densité linéaire (PEBDL), de copolymères ioniques, de caoutchouc naturel, de caoutchouc synthétique, d'élastomères, et de polyéthylène haute densité (PEHD).

4. Procédé selon la revendication 3, ledit acrylate d'alkyle comprenant au moins l'un parmi l'acrylate de méthyle et l'acrylate d'éthyle.

5. Procédé selon l'une quelconque des revendications précédentes, ledit matériau en mousse à alvéoles ouvertes comprenant un élément parmi l'éthylène-acrylate de méthyle (EMA), l'éthylène-acrylate de vinyle (EVA), l'éthylène-acrylate d'éthyle (EEA), l'éthylène-acrylate de butyle (EBA), le polyéthylène basse densité (PEBD), le polyéthylène basse densité linéaire (PEBDL), le polyéthylène très basse densité (PETBD), le polyéthylène haute densité (PEHD), le polypropylène (PP), le caoutchouc naturel, le monomère éthylène-propylène-diène (EPDM), le caoutchouc synthétique, le polyéthylène chloré (CPE), les copolymères séquencés d'oléfines, le copolymère éthylène-anhydride maléique, les polyoléfines à amorçage en un site unique, les polyoléfines catalysées par métallocène, les polymères greffés comprenant notamment mais non exclusivement le silane et l'anhydride maléique, les copolymères styrène-butadiène-styrène, le polyisoprène, et les équivalents et les mélanges de ceux-ci.

6. Procédé selon l'une quelconque des revendications précédentes, lesdites substances étant choisies dans le groupe des substances constituées par le pétrole, les composés organiques de la gamme diesel, les composés organiques de la gamme essence, les fluides de forage, les biocides, le glutaraldéhyde, les métaux, les composés organométalliques, les métalloïdes, les composés organiques volatiles (COV), les composés organiques semi-volatiles (COSV), les pesticides, les biphényles polychlorés (PCB), les engrais, les solvants, les déchets humains, les produits pharmaceutiques, les matières radioactives et les composants de ceux-ci.

7. Procédé selon l'une quelconque des revendications précédentes, ledit matériau en mousse à alvéoles ouvertes comprenant une pluralité de structures distinctes choisies dans le groupe des structures constituées de bandes, de cubes et de petites pièces, lesdites structures distinctes étant maintenues en place par un ou plusieurs éléments parmi un dispositif d'ancrage, un poids, un filet et un récipient doté d'ouvertures destinées à permettre l'écoulement d'eau à travers celui-ci.

8. Procédé selon l'une quelconque des revendications précédentes, ledit placement du matériau en mousse à alvéoles ouvertes comprenant la mise en suspension d'une pluralité de structures distinctes à différents niveaux à travers une hauteur de colonne d'eau, comprenant la flottaison d'une structure au moins partiellement à la surface de l'eau.

9. Procédé selon l'une quelconque des revendications précédentes, ledit second temps d'exposition étant d'au moins huit heures.

10. Procédé selon la revendication 1, ledit placement du matériau en mousse à alvéoles ouvertes comprenant le lancer du matériau en mousse à alvéoles ouvertes dans le corps d'eau avec une canne à pêche.

11. Procédé selon la revendication 1, ledit placement du matériau en mousse à alvéoles ouvertes comprenant le fait de tracter le matériau en mousse à alvéoles ouvertes derrière un bateau qui se déplace à travers le corps d'eau, ou la flottaison du matériau à alvéoles ouvertes à la surface du corps d'eau, ou le couplage du matériau en mousse à alvéoles ouvertes à un quai, ou le placement du matériau en mousse à alvéoles ouvertes dans une baignoire ou un évier.
